# EUROPEAN PATENT APPLICATION

(11) **EP 1 093 756 A2**
(43) Date of publication of application: **25.04.2001**
(21) Application number: 00122633.1
(22) Date of filing: 17.10.2000
(51) Int. Cl.: A61B 10/00

(54) **Sample cell, and solution concentration measuring apparatus and method of urinalysis using the same**

(30) Priority: 18.10.1999 JP 29566299
(71) Applicant: Matsushita Electric Industrial Co., Ltd., Kadoma-shi, Osaka 571-8501 (JP)
(72) Inventor: Kawamura, Tatsurou, Kyotanabe-shi, Kyoto 610-0351 (JP)
(74) Representative: Leson, Thomas Johannes Alois, Dipl.-Ing.

(57) **Abstract**

An object of the present invention is to eliminate a need for weighing a solution to be detected, thereby achieving higher efficiency and laborsaving of a measurement and a test, and to provide a sample cell having a configuration capable of holding the solution, and irradiating the solution with light, the sample cell, comprising: an opening for introducing the solution, and a reagent on the top side of the sample cell; and an outlet port capable of discharging a surplus solution in excess of a prescribed amount between a light propagation area in the holding portion of a sample and the opening, and a concentration measuring apparatus and a urinalysis apparatus using the same.

## Description

### BACKGROUND OF THE INVENTION

The present invention relates to a sample cell for use in determination of the concentration of a solution to be detected, and an operating method thereof. More particularly, it relates to an apparatus for measuring the concentration of a solution, and a urinalysis apparatus, using the same.

When the concentration of the solution to be detected is determined by injecting a reagent into the solution to be detected, the volume ratio between the solution to be detected and the reagent is required to be fixed or controlled. With the sample cell of the present invention, the volume ratio can be fixed or controlled with ease. Especially when the solution to be detected is a urine, the urine can be directly discharged into the sample cell. Therefore, ease and high reliability of the urinalysis, and the compactness and lower price of the urinalysis apparatus can be achieved, resulting in high practical utility.

In general, when the optical characteristics of a substance to be detected contained in a solution to be detected is determined, thereby to calculate the concentration therein, the solution to be detected is held by a sample cell. The sample cell is so configured that light propagates through the inside of the solution held thereby. For example, the sample cell is configured with glass or the like, and has the form of a cube (rectangular parallelepiped), and the light-transmitting surface thereof is transparent.

Such a sample cell is open at the top, through which a solution to be detected is introduced in a prescribed amount by a dropper, a pipette or a syringe. Then, a reagent is introduced thereinto in a prescribed amount, thereby making the volume ratio between the solution to be detected and the reagent constant. Thus, the optical characteristics of the substance to be detected in the solution to be detected is measured to determine the concentration.

However, with the sample cell employing such a configuration, a prescribed amount of a solution to be detected is required to be introduced into the sample cell for making the volume ratio between the solution to be detected and the reagent constant. For this reason, there is required a step of previously charging the solution to be detected into a beaker or the like, and weighing it by a pipette or a syringe to determine the volume, and then introducing the solution to be detected into the sample cell. This step also presents a problem of not only complicating the concentration measurement of the solution to be detected, but also making an error due to a mistake during the weighing operation more likely to occur.

Further, when the solution to be detected is a urine, there is a need for weighing the urine once discharged into a cup or the like, and then introducing it in a prescribed amount into the sample cell. This also presents another problem that the step is troublesome especially when the urinalysis is carried out at home, causing a user to have a great repugnance.

In view of the foregoing problems in the prior art, it is therefore an object of the present invention to provide a sample cell which does not require previous weighing of the volume of a solution to be detected in introducing the solution to be detected into the sample cell. In other words, it is an object of the present invention to provide a sample cell whereby upon charging a solution to be detected in an amount equal to or more than a prescribed amount into the sample cell, the solution to be detected in only an amount required for the subsequent measurement remains in the sample cell.

If such a sample cell can be implemented, only by fixing or controlling the volume of the reagent required for the measurement of the solution to be detected without previously weighing the solution to be detected, it is possible to fix or control the mixing ratio thereof.

Namely, the present invention provides a sample cell which requires no weighing of a solution to be detected, ensures a very great practical effect, and enables greater efficiency and laborsaving of a measurement and a test, and a concentration measuring apparatus using the same.

### BRIEF SUMMARY OF THE INVENTION

The present invention mainly provides two types of sample cells for solving the foregoing problems.

A first sample cell is a sample cell having a holding portion capable of holding a solution to be detected and irradiating the solution to be detected with light, which comprises: an opening for introducing the solution to be detected and a reagent on the top side of the sample cell; and an outlet port capable of discharging a surplus solution to be detected in excess of a prescribed amount between a light propagation area in the holding portion and the opening.

In this case, it is effective that the opening is in funnel form.

It is also effective that the sample cell further comprises an inlet port for injecting the reagent at the lower part than the light propagation area in the holding portion.

Further, it is also effective that the sample cell comprises: an optical entrance window for irradiating the solution to be detected with light; a first optical exit window for emitting the light transmitted through the solution to be detected; and a second optical exit window for emitting scattered light arising from the solution to be detected, wherein the outlet port is located at a portion other than a portion having the optical window.

The present invention also provides a concentration measuring apparatus of a solution, which comprises: a light source for projecting light on a solution to be detected; the above-mentioned first sample cell for holding the solution to be detected; a photosensor for detecting the light transmitted through the solution to be detected and/or the scattered light arising from the solution to be detected; an introduction means for introducing the solution to be detected into the sample cell; an injection means for injecting the reagent into the sample cell; and a computer for controlling the introduction means and the injection means to analyze the optical characteristics of the solution to be detected based on an output signal from the photosensor.

In this case, it is effective that the light source, the sample cell, and the photosensor are integrally accommodated in a sealed (air-tightly closed) enclosure or cabinet, and the opening of the sample cell is open to the outside.

Then, the present invention also pertains to a method of urinalysis using the concentration measuring apparatus comprising the first sample cell, the method comprising the steps of: directly discharging a urine which is a solution to be detected from the opening of the sample cell into the holding portion; injecting a reagent from the opening or the inlet port; projecting light on the urine; and determining the transmitted light and/or the scattered light of the urine to examine the urine.

Then, a second sample cell of the present invention is a sample cell having a holding portion capable of holding a solution to be detected and irradiating the solution to be detected with light, which comprises: an opening for introducing the solution to be detected, and a reagent on the top side of the sample cell; and an inclining (tilting) means for inclining the sample cell.

It is also effective in this case that the opening is in funnel form.

Further, it is also effective that the sample cell further comprises an inlet port for injecting the reagent at the lower part than the light propagation area in the holding portion.

Still further, it is also effective that the sample cell comprises: an optical entrance window for irradiating the solution to be detected with light; a first optical exit window for emitting the light transmitted through the solution to be detected; and a second optical exit window for emitting the scattered light arising from the solution to be detected, and the outlet port is located at a portion other than the portion having the optical window.

Further, the present invention also pertains to a concentration measuring apparatus of a solution, which comprises: a light source for projecting light on a solution to be detected; the above-mentioned second sample cell for holding the solution to be detected; a photosensor for detecting the light transmitted through the solution to be detected and/or the scattered light arising from the solution to be detected; an introduction means for introducing the solution to be detected into the sample cell; an injection means for injecting the reagent into the sample cell; and a computer for controlling the inclining means, the introduction means, and the injection means to analyze the optical characteristics of the solution to be detected based on an output signal from the photosensor.

In this case, it is effective that the light source, the sample cell, and the photosensor are integrally accommodated in a sealed enclosure or cabinet, and the opening of the sample cell is open to the outside.

Still further, the present invention also pertains to a method of urinalysis using the concentration measuring apparatus comprising the second sample cell, the method comprising the steps of: directly discharging a urine which is a solution to be detected from the opening of the sample cell into the holding portion; discharging the surplus solution to be detected in excess of a prescribed amount through said opening by inclining the sample cell by the inclining means; injecting a reagent from the opening or the inlet port; and determining the transmitted light and/or scattered light of the urine to examine the urine.

While the novel features of the invention are set forth particularly in the appended claims, the invention, both as to organization and content, will be better understood and appreciated, along with other objects and features thereof, from the following detailed description taken in conjunction with the drawings.

### BRIEF DESCRIPTION OF THE SEVERAL VIEWS OF THE DRAWING

FIG. 1 is a perspective view of a sample cell in accordance with one embodiment of the present invention;
FIG. 2 is a side elevation view of the sample cell shown in FIG. 1;
FIG. 3 is a top plan view of the sample cell shown in FIG. 1;
FIG. 4 is a schematic view showing a configuration of a concentration measuring apparatus in accordance with Example 1 of the present invention;
FIG. 5 is a schematic view showing a configuration of a concentration measuring apparatus in accordance with Example 2 of the present invention;
FIG. 6 is a top plan view of a sample cell in the concentration measuring apparatus shown in FIG. 5;
FIG. 7 is a schematic view showing a configuration of a concentration measuring apparatus in accordance with Example 3 of the present invention;
FIG. 8 is a schematic view showing a configuration of a concentration measuring apparatus in accordance with Example 4 of the present invention;
FIG. 9 is a schematic view showing a configuration of a concentration measuring apparatus in accordance with Example 5 of the present invention;
FIG. 10 is a schematic view showing a configuration of a concentration measuring apparatus in accordance with Example 6 of the present invention; and
FIG. 11 is a partly sectional schematic top plan view of the concentration measuring apparatus shown in FIG. 10.

### DETAILED DESCRIPTION OF THE INVENTION

The present invention pertains to a sample cell having a configuration capable of holding a solution to be detected (hereinafter, referred to as "solution), and irradiating the solution with light, which comprises an opening for introducing the solution and a reagent at the top side of the sample cell, and an outlet port capable of discharging the surplus solution to be detected in excess of a prescribed amount between a light propagation area in the holding portion of the solution and the opening.

In concrete, by providing the outlet port at a predetermined portion to determine the height of the outlet port from the holding portion of the sample cell, in other words, by predetermining a volume of the sample cell below the outlet port, the sample cell can hold a specified amount of solution without any weighing procedure.

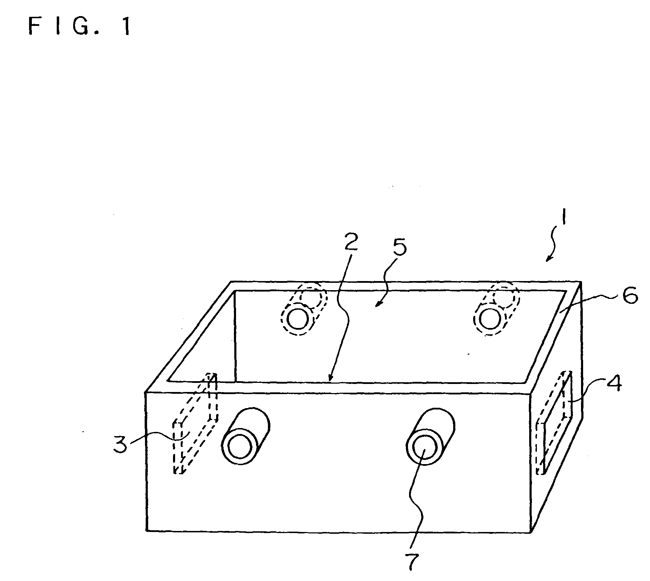

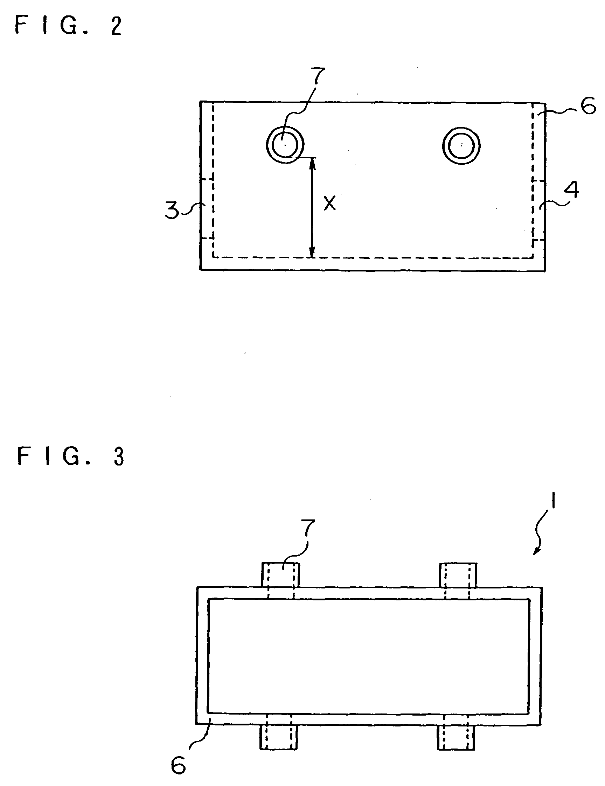

The basic configuration of the present invention will be described by reference to drawings. FIG. 1 is a perspective view of a sample cell in accordance with one embodiment of the present invention. Whereas, FIG. 2 is a side elevation view of the sample cell of the present invention as seen from the direction of an arrow in FIG. 1.

As shown in FIG. 1, a sample cell 1 of the present invention has a holding portion 2 for a solution, and has an opening 5 for introducing the solution and a reagent on the top side thereof. Further, the sample cell 1 has, for example, an optical entrance window 3 for irradiating the solution with light, and an optical exit window 4 for emitting the light transmitted through the solution as the configuration capable of irradiating the solution with light.

As for the shape of a skeleton 6 of the sample cell 1, it is assumed to be a rectangular parallelepiped in FIGS. 1 and 2. However, it is not limited thereto so long as it is within the spirit of the present invention. For example, the shapes such as a cube, a cylinder and a prism may also be acceptable. Out of these, a rectangular parallelepiped including a cube is preferred in that the sample cell itself can be disposed with stability.

Further, as materials for constituting the skeleton 6 of the sample cell 1, mention may be made of, for example, metals such as aluminum and copper, glass, and resins. When the temperature control of the sample cell is required, a metal with high heat conductivity is advantageous. Out of these, aluminum is preferably used in terms of processability and cost.

Then, as the optical windows 3 and 4, optical windows made of glass plate conventionally used can be used, and they have no particular restriction so long as they fall in such a range as not to impair the effect of the present invention.

Further, as for the positions for respectively disposing the optical windows 3 and 4, the optical windows 3 and 4 may be disposed at two different sidewalls in the skeleton of the sample cell. As shown in FIGS. 1 and 2, if the optical windows 3 and 4 are respectively disposed at the two opposing sides, it is possible to irradiate the solution contained in the holding portion 2 with light from the optical entrance window 3, and to take the transmitting light out of the optical exit window 4. When the skeleton shape of the sample cell is the shape other than a rectangular parallelepiped, the position of the optical windows may be determined according to the shape while ensuring the path for the light transmitting through the solution in the sample cell.

Here, it is effective that the opening 5 is in a funnel form from the viewpoint of introducing the solution and the reagent into the holding portion 2 of the sample cell 1 with reliability. Therefore, the present invention provides a sample cell having a configuration capable of holding a solution to be detected, and irradiating the solution with light, which comprises an opening for introducing the solution and a reagent therein at the top side of the sample cell, the opening being in a funnel form.

The greatest feature of the sample cell of the present invention is in that an outlet port 7 for allowing the surplus solution in excess of a prescribed amount to escape therethrough is provided between the light propagation area in the holding portion 2 and the opening 5 as shown in FIGS. 1 and 2.

The outlet port 7 has such a function as to let out the surplus solution in excess of the prescribed amount from the holding portion 2. Therefore, the outlet port 7 must be disposed at such a position that the foregoing function can be exerted in the sample cell 1.

Namely, as shown in FIG. 2, if the outlet port 7 is provided at a position of distance X from the bottom in the sample cell 1, it is possible to make constant the amount of the solution to be left in the holding portion 2 by adjusting the distance X while fixing the volume of the holding portion 2 of the sample cell 1.

According to the sample cell of the present invention, thus, by adjusting the position of the outlet port 7 to make the amount of the solution to be detected held in the holding portion 2 constant, it becomes sufficient to weigh only the amount of the reagent when the solution is mixed with the reagent required for the concentration of the solution to be detected.

Here, the outlet port 7 may be made of the same material as the material for constituting the skeleton of the sample cell 1. Of course, it is also possible to manufacture it with a different material from the material for constituting the skeleton of the sample cell 1.

It is noted that, although the example in which four outlet ports 7 are provided is shown in FIGS. 1 and 2, there is no particular restriction as to the number thereof so long as the effect of the invention is not impaired.

Then, in the sample cell shown in FIGS. 1 and 2, both of the solution and the reagent can be introduced through the opening 5 into the holding portion 2. However, for avoiding a problem that the proportion of the reagent to be partially discharged with the solution increases when the proportion of the reagent is increased, it is preferable that an inlet port for introducing the reagent is provided separately from the opening 5.

Further, the inlet port is preferably located at the lower position than the light propagation area in the holding portion 2. The reason for this is that by increasing the distance between the inlet port and the outlet port or the opening, it is possible to control the increasing rate in proportion of the reagent to be partially discharged with the solution when the proportion of the reagent is large. Another reason is that there is an effect of collecting bubbles, which have been generated and mixed therein upon introducing the reagent, at the upper portion by forming an upward flow, resulting in a prevention of the bubbles from interrupting the measurement or determination.

The sample cell of the present invention includes, in addition to the optical entrance window 3 for irradiating the solution with light, and the optical exit window 4 for emitting the light transmitted through the solution, another (second) optical exit window for emitting the scattered light arising from the solution.

Further, when these three types of optical windows are provided especially in a sample cell having the form of a rectangular parallelepiped, the outlet port may be located at a sidewall other than the sidewalls respectively having the optical windows.

Still further, it is effective that the sample cell of the present invention comprising an inclining means for inclining the sample cell itself. The reason for this is as follows. When the outlet port is provided, it is possible to allow a prescribed amount of the solution to remain in the holding portion of the sample cell. However, there may occur the cases where there is no outlet port; the outlet port is clogged for some reason; the amount of the reagent usable is limited; there arises a need to exhaust the solution in an amount equal to or larger than the amount of the solution discharged through the outlet port; and the like.

Namely, the present invention provides a sample cell having a configuration capable of holding a solution to be detected and irradiating the solution with light, which comprises an opening for introducing the solution and a reagent at the top side of the sample cell, and an inclining means for inclining the sample cell.

Any inclining means is acceptable so long as it has a function of discharging the solution in the holding portion by inclining the sample cell. Such a function can be attained, for example, by providing a shaft to the skeleton of the sample cell, and rotating the shaft by a motor, as described in an example below.

The present invention also pertains to an operating method of the foregoing sample cell.

As described above, the sample cell of the present invention may be used by a method whereby the function can be performed. For example, the sample cell having an inclining means of the present invention can be operated by (a) introducing a solution to be detected from the opening into the holding portion, (b) discharging the surplus solution in excess of the prescribed amount by inclining the sample cell with the inclining means, and then (c) injecting a reagent from the opening or the inlet port.

Especially when the inlet port is provided in addition to the opening in the sample cell, as described above, the reagent is preferably injected from the inlet port in the step (c).

The sample cell of the present invention as described above can be preferably used for measurement of the concentration of a substance to be detected in a solution to be detected. Therefore, the present invention can also provides an apparatus for measuring the concentration of a solution.

Namely, the present invention also pertains to an apparatus for measuring the concentration of a solution, which comprises: a light source for projecting light on a solution to be detected; the sample cell for holding the solution; a photosensor for detecting the light transmitted through the solution and/or the scattered light arising from the solution; an introduction means for introducing the solution into the sample cell; an injection means for injecting the reagent into the sample cell; and a computer for controlling the introduction means and the injection means to analyze the optical characteristics of the solution based on an output signal from the photosensor.

It is noted that the optical characteristics herein used denotes scattering, transmission, fluorescence, optical rotation, polarization and the like.

Whereas, the introduction means is not restricted if it can introduce the solution into the sample cell. Examples of the introduction means usable include funnel and electromagnetic valve, pump, and the like. Further, the injecting means is not restricted if it can inject the reagent into the sample cell. Examples of the reagent injection means usable include pipette, syringe, pump and the like.

Especially when the sample cell has the inclining means, it is effective that the computer is allowed to control the inclining means, the introduction means, and the injection means to analyze the optical characteristics of the solution based on an output signal from the photosensor.

Further, each component of the concentration measuring apparatus herein referred to can be appropriately selected except for the sample cell in order to achieve the effect of the invention by any those skilled in the art.

Further, the concentration measuring apparatus preferably has such a structure that the light source, the sample cell, and the photosensor are integrally accommodated in a sealed enclosure. However, the opening of the sample cell is required to be open to the outside.

Although the enclosure can also be made of the materials for constituting the sample cell, it is preferably made of a resin from the viewpoint of easy handling. Further, if the resin is used, there is particularly an advantage in that each component is easily enclosed and fixed. Therefore, it is also possible to cover each component with a resin for fixing it.

Namely, if the enclosure is used, the positional relation between the light source, the sample cell, and the photosensor relative to one another can be fixed. Consequently, the apparatus can be moved together with the enclosure to the position where the solution can be introduced into the sample cell with ease. This eliminates the necessity to conduct the optical alignment of optical axes and the like for each concentration measurement, resulting in no error of measurement.

Further, since the enclosure can be placed at the right position to directly supply the solution to the concentration measuring apparatus, it is possible to promote the use of the apparatus. Still further, since the enclosure has a sealed structure, it is possible to eliminate the possibility that the solution and the reagent contaminate respective optical components constituting the concentration measuring apparatus to obstruct the measurement. Thus, a malfunction is less likely to occur, and excellent practical effects can be exerted, and hence the greater efficiency and laborsaving in the concentration measurement can be attained.

Such a concentration measuring apparatus can be applied to a urinalysis apparatus by using a urine as the solution to be detected. In other words, the present invention also provides a urinalysis apparatus.

Namely, the present invention also provides a urinalysis apparatus comprising the concentration measuring apparatus for detecting a urine by performing urination directly toward the sample cell, and thereby introducing the urine into the holding portion for the solution to be detected in the sample cell.

Here, the present invention also pertains to a method of urinalysis using the concentration measuring apparatus.

Namely, the present invention provides a method of urinalysis using the concentration measuring apparatus, which comprises the steps of: directly discharging a urine which is a solution to be detected from the opening of the sample cell into the holding portion; injecting a reagent from the opening or the inlet port; projecting light on the urine; and determining the transmitted light and/or scattered light of the urine to examine the urine.

Especially when a sample cell having an inclining means is used, there is provided a method of urinalysis using the concentration measuring apparatus, which comprises the steps of: directly discharging a urine which is a solution to be detected from the opening of the sample cell into the holding portion; discharging the surplus solution in excess of a prescribed amount from the opening by inclining the sample cell with the inclining means; injecting a reagent from the opening or the inlet port; and determining the transmitted light and/or scattered light of the urine to examine the urine.

It is noted that, in the above description, the functions of the sample cell of the present invention were individually described. However, a sample cell having a plurality of functions can also be obtained so long as it does not impair the effects of the present invention.

Namely, it is also possible to obtain a sample cell having an appropriate combination of an outlet port, an inclining means and/or a funnel-like opening.

Below, the sample cell of the present invention and the concentration measuring apparatus using the same will be described concretely by way of the following examples, which should not be construed as limiting the scope of the invention.

### Example 1

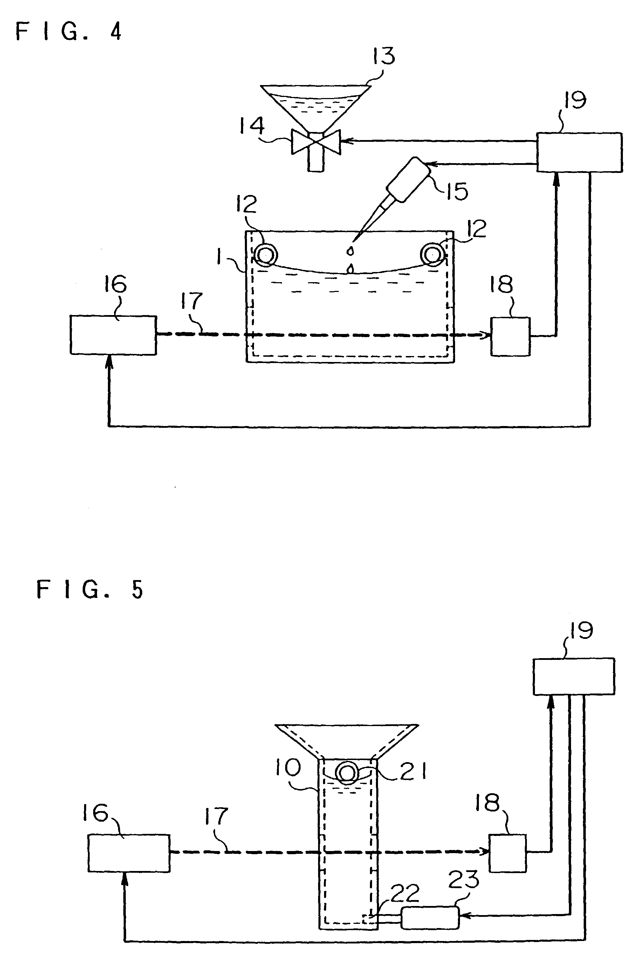

First, Example 1 of the present invention will be described by reference to FIG. 4. FIG. 4 is a schematic view showing a configuration of a concentration measuring apparatus in accordance with the present invention.

In FIG. 4, a sample cell 1 of the present invention is a container made of aluminum in the form of a rectangular parallelepiped having an opening open upwards, wherein glass plates are embedded as optical windows at the opposite ends of an optical path, so that light can transmit through the inside of a solution to be detected while the solution being held. The length along the major axis direction, i.e., the distance between the optical windows of the container is 50 mm, while the length along the minor axis direction thereof is 10 mm. Whereas, respective outlet ports 12 disposed at both ends of the sides having no optical window are located at the same height from the bottom. The lowermost part of the opening of each outlet port 12 is situated at a height of 20 mm (X in FIG. 2) from the bottom of the holding portion of the sample cell. Therefore, 10 ml of the solution can be constantly held in the sample cell 1.

Further, the apparatus in accordance with this embodiment includes a funnel 13 for temporarily trapping the solution, an electromagnetic valve 14 for controlling the dropping of the solution trapped by the funnel 13 into the sample cell 1, a pipette 15 for injecting a prescribed amount of a reagent into the solution, and a semiconductor laser module 16 serving as a light source, and projects a substantially parallel light 17 with a wavelength of 780 nm, an intensity of 3.0 mW, and a beam diameter of 2.0 mm. Further, it has a computer 19 for analyzing an output signal from a photosensor 18 for detecting the light transmitted through the solution, to control the electromagnetic valve 14, the pipette 15, and the light source 16.

The operation of the apparatus in accordance with this Example is as follows.

First, the solution is charged into the trapping funnel 13, and the computer 19 controls the electromagnetic valve 14 to introduce the solution trapped by the funnel 13 into the sample cell 1. The solution may be introduced in any amount at this step so long as it is in an amount equal to or more than a prescribed amount, i.e., 10 ml. The reason for this is that the surplus solution in excess of 10 ml is discharged through the outlet port 12. At this step, since the outlet ports are disposed at the sides having no optical window, the discharged solution does not adhere to the optical windows to obstruct the measurement.

Then, the computer 19 controls the pipette 15 to inject 0.5 ml of the reagent into the sample cell 1. In this step, the solution in the sample cell 1 is discharged in an amount of about 0.5 ml through the outlet port 12. Here, the computer 19 analyzes the output signal from the photosensor 18 to determine the concentration of the solution.

As described above, according to this Example, there is no need to previously weigh the volume of the solution to be detected, and by introducing the solution in an amount equal to or larger than the prescribed amount, it is possible to fix or control the volume ratio with the reagent to be injected thereinto. Consequently, the process can be simplified, and further a malfunction is less likely to occur. Accordingly, the practical effect thereof is very great, and higher efficiency and laborsaving of the measurement and the test become possible.

### Example 2

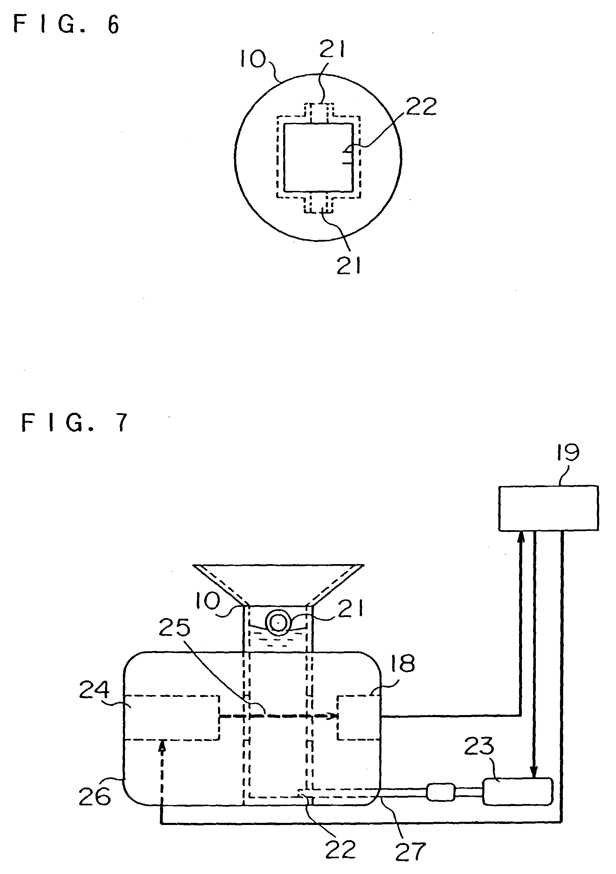

Example 2 will be described in details below by reference to FIGS. 5 and 6. FIG. 5 is a schematic view showing a configuration of a concentration measuring apparatus in accordance with Example 2 of the present invention. Whereas, FIG. 6 is a top plan view of a sample cell in the concentration measuring apparatus shown in FIG. 5.

In FIGS. 5 and 6, the light source 16, the substantially parallel light 17, the photosensor 18, and the computer 19 are the same as those shown in FIG. 4. A sample cell 10 is a container made of aluminum in the form of a rectangular parallelepiped having an opening portion in funnel form at the top thereof as described in the figure, wherein glass plates are embedded as optical windows at the opposite sides of an optical path, so that light can transmit through the inside of a solution while the solution being held. The length along the direction of propagation of light in the container, i.e., the distance between the optical windows of the container is 10 mm, while the length thereof along the direction perpendicular to the propagation direction is 10 mm. In the sample cell 10, respective outlet ports 21 disposed at both ends of the sides having no optical window are located at the same height from the bottom. The underside of each outlet port 21 is situated at a height of 30 mm from the bottom. Therefore, 30 ml of the solution can be constantly held in the sample cell 10. Further, an inlet port 22 for injecting a reagent therein is located at the bottom of the sample cell 10, i.e., at the lower part of the light propagation area. A pipette 23 for injecting a prescribed amount of the reagent through the inlet port 22 into the solution in the sample cell 10 is controlled by the computer 19.

The operation of the apparatus in accordance with this Example is as follows.

First, the solution is charged into the sample cell 10. The solution may be introduced therein in any amount at this step so long as it is in an amount equal to or more than a prescribed amount, i.e., 3 ml. The reason for this is that the surplus solution in excess of 3 ml is allowed to escape through the outlet port 21. At this step, since the outlet ports are disposed at the sides having no optical window, the discharged solution does not adhere to the optical window to obstruct the measurement.

Then, the computer 19 controls the pipette 23 to inject 1.5 ml of a reagent into the sample cell 10. In this step, about 15 ml of the solution in the sample cell 10 is discharged through the outlet ports 21. Here, the computer 19 analyzes the output signal from the photosensor 18 to determine the concentration of the solution.

As described above, according to this Example, there is no need to previously weigh the volume of the solution, and by introducing the solution in an amount equal to or larger than the prescribed amount, it is possible to fix or control the volume ratio with the reagent to be injected thereinto. In this Example, the reagent is injected from the bottom of the sample cell 10, and the outlet ports 21 are located at the upper part of the sample cell 10. Therefore, the proportion of the solution to be replaced with the reagent is higher than with Example 1, and hence the apparatus operates with stability even in the case where the volume ratio of the reagent is large. Consequently, the process can be simplified, and further a malfunction is less likely to occur, as well as the operational stability is improved. Accordingly, the practical effect thereof is very great, and higher efficiency and laborsaving of the measurement and the test become possible.

### Example 3

Example 3 will be described in details below by reference to FIG. 7. FIG. 7 is a schematic view showing a configuration of a concentration measuring apparatus in accordance with this example.

In FIG. 7, the sample cell 10, the photosensor 18, the computer 19, the outlet ports 21, the inlet port 22, and the pipette 23 are the same as those shown in FIGS. 5 and 6 in Example 2. This apparatus has a light emitting diode projector module 24 as a light source, and projects a substantially parallel light 25 with a center wavelength of 540 nm, an intensity of 0.1 mW, and a beam diameter of 2.0 mm. An enclosure 26 is one made of a resin, which integrates the sample cell 10, the photosensor 18, the outlet ports 21, the inlet port 22, and the light source 24 out of these components into one piece, and has a sealed structure. Consequently, even if the solution splashes on the enclosure, it does not reach the photosensor 18, the light source 24, and the optical windows on the outside of the sample cell 10. Further, since the solution discharged through the outlet ports 21 is also covered with the enclosure 26, the measurement can be prevented from being obstructed. A tube 27 for transferring the reagent connects the pipette 23 with the inlet port 22, so that the reagent is injected in a prescribed amount through the tube 27 under the control of the computer 19.

When the apparatus in accordance with this Example is used to detect the protein concentration using a urine as a solution to be detected, the operation thereof is as follows.

First, the apparatus is transferred to the inside of a lavatory and the like where a urine, which is a solution to be detected, is easily trapped together with the enclosure 26. Here, a subject directly discharges a urine into the sample cell 10. The urine may be discharged in any amounts into the funnel-like opening of the sample cell 10 so long as it is in an amount equal to or more than a prescribed amount, i.e., 3 ml. The reason for this is that the surplus solution in excess of 3 ml is discharged through the outlet ports 21. At this step, since the apparatus is covered with the enclosure 26, the discharged solution does not adhere to the optical windows to obstruct the measurement.

Then, the computer 19 controls the pipette 23 to inject 1.5 ml of a biuret reagent (a reagent obtained by dissolving potassium sodium tartrate and copper sulfate in a sodium hydroxide solution) into the sample cell 10. In this step, the solution to be detected is discharged in an amount of about 1.5 ml through the outlet ports 21. Here, the computer 19 analyzes the output signal from the photosensor 18 to determine the concentration of the solution to be detected.

As described above, according to this Example, there is no need to previously weigh the volume of the solution to be detected, and by introducing the solution in an amount equal to or larger than the prescribed amount, it is possible to fix or control the volume ratio with the reagent to be injected thereinto. In this Example, the reagent is injected from the bottom of the sample cell 10, and the outlet ports 21 are located at the upper part of the sample cell 10. Therefore, the proportion of the solution to be replaced with the reagent is higher than with Example 1, and hence the apparatus operates with stability even in the case where the volume ratio of the reagent is large.

Further, the apparatus is easily moved together with the enclosure 26 to the right place for trapping the solution to be detected. In this step, as compared with the case where only the sample cell is transferred, an optical alignment error of optical axes, and the like does not occur. Further, since the enclosure 26 has a sealed structure, there is no danger that the solution to be detected, and the like adhere to respective optical components to obstruct the measurement. Consequently, the urinalysis can be performed with ease, and further a malfunction is less likely to occur, as well as the operational stability is improved. Accordingly, the practical effect thereof is very great, and higher efficiency and laborsaving of the measurement and the test become possible.

### Example 4

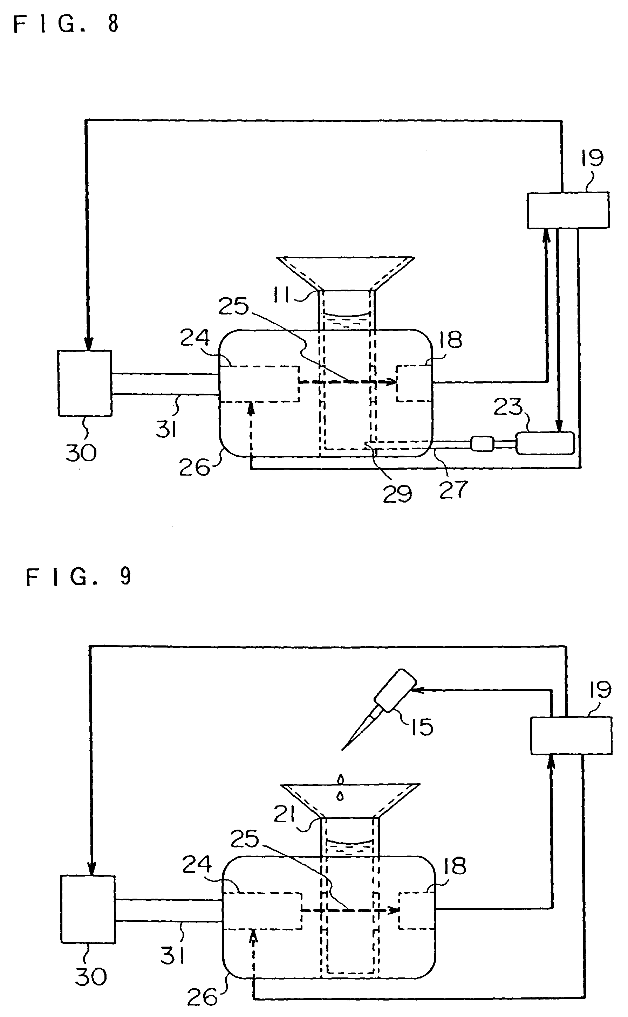

Example 4 will be described in details below by reference to FIG. 8. FIG. 8 is a schematic view showing a configuration of a concentration measuring apparatus in accordance with this example.

In FIG. 8, the photosensor 18, the computer 19, the pipette 23, the light source 24, the substantially parallel light 25, the enclosure 26, and the tube 27 are the same as those shown in FIG. 7 in Example 3. A sample cell 11 has the form of the sample cell 10 in FIG. 7 having no outlet port, and the prescribed amount is also similarly 3 ml. A reagent inlet port 29 has the same form as that of the inlet port 22 installed at the sample cell 11 in FIG. 7. As with Example 3, the sample cell 11, the photosensor 18, the light source 24, and the inlet port 29 are integrated into one piece by the enclosure 26 made of a resin. A motor 30 rotates in the direction parallel to the paper plane of the figure through a rotational shaft 31, thereby allowing the enclosure 26 to be inclined under the control of the computer 19. The tube 27 for transferring the reagent connects the pipette 23 with the inlet port 29, so that the reagent is injected therein in a prescribed amount through the tube 27 under the control of the computer 19.

When the apparatus in accordance with this Example is used to detect the protein concentration using a urine as a solution to be detected, the operation thereof is as follows.

First, the apparatus is transferred to the inside of a lavatory or the like where the urine, which is a solution to be detected, is easily trapped together with the enclosure 26. Here, a subject directly discharges a urine into the sample cell 11. Then, the computer 19 provides a direction, so that the enclosure 26 is inclined with respect to the shaft 31 as an axis to discharge the solution through the funnel-like opening at the top thereof. By adjusting the angle of inclining at this step, it is possible to control the volume of the solution remaining in the sample cell 11. In this example, it is set such that the half of the prescribed amount, i.e., 1.5 ml of the solution remains therein. Therefore, the urine may be discharged in any amounts into the funnel-like opening of the sample cell 11 so long as it is in an amount equal to or more than 1.5 ml. At this step, since the apparatus is covered with the enclosure 26, the discharged solution does not adhere to the optical window, and the like to obstruct the measurement.

Then, the computer 19 controls the pipette 23 to inject 1.5 ml of a biuret reagent (a reagent obtained by dissolving potassium sodium tartrate and copper sulfate in a sodium hydroxide solution) into the sample cell 11. Here, the computer 19 analyzes the output signal from the photosensor 18 to determine the concentration of the solution to be detected.

As described above, according to this Example, there is no need to previously weigh the volume of the solution, and by introducing the solution in an amount equal to or larger than the prescribed amount, it is possible to fix or control the volume ratio with the reagent to be injected thereinto. In this Example, only a prescribed amount of the solution to be detected is allowed to remain in the sample cell 11, and a prescribed amount of the reagent is injected, thereby fixing or controlling the volume ratio. Since this process does not involve the replacement as with Examples 1 to 3, the apparatus operates with stability even in the case where the volume ratio of the reagent is large.

Further, the apparatus is easy to transfer together with the enclosure 26 to the right place for trapping the solution to be detected, and hence it is effective. In this step, as compared with the case where only the sample cell is transferred, an optical alignment error of optical axes, and the like does not occur. Further, since the enclosure 26 has a sealed structure, there is no danger that the solution, and the like adhere to respective optical components to obstruct the measurement. Consequently, the urinalysis can be performed with ease, and further a malfunction is less likely to occur, as well as the operational stability is improved. Accordingly, the practical effect thereof is very great, and higher efficiency and laborsaving of the measurement and the test become possible.

### Example 5

Example 5 will be described in details below by reference to FIG. 9. FIG. 9 is a schematic view showing a configuration of a concentration measuring apparatus in accordance with this example.

In FIG. 9, the photosensor 18, the computer 19, the light source 24, the substantially parallel light 25, the enclosure 26, the motor 30, and the shaft 31 are the same as those shown in FIG. 8 in Example 4. A sample cell 20 has the form of the sample cell 11 in FIG. 8 having no inlet port, and the prescribed amount is also similarly 3 ml. As with Example 3, the photosensor 18, the light source 24, and the sample cell 20 are integrated into one piece by the enclosure 26 made of a resin. The pipette 15 is the same as that used in Example 1, and controlled by the computer 19 to inject a prescribed amount of a reagent into the sample cell 20.

When the apparatus in accordance with this Example is used to detect the protein concentration using a urine as a solution to be detected, the operation thereof is as follows.

First, the apparatus is transferred to the inside of a lavatory or the like where the urine, which is a solution to be detected, is easily trapped together with the enclosure 26. Here, a subject directly discharges a urine into the sample cell 20. Then, the computer 19 provides a direction, so that the enclosure 26 is inclined with respect to the shaft 31 as an axis to discharge the solution through the funnel-like opening at the top thereof. By adjusting the angle of inclining at this step, it is possible to control the volume of the solution remaining in the sample cell 20. In this example, it is set such that the half of the prescribed amount, i.e., 1.5 ml of the solution remains therein. Therefore, the solution may be discharged in any amounts into the funnel-like opening of the sample cell 20 so long as it is in an amount equal to or more than 1.5 ml. At this step, since the apparatus is covered with the enclosure 26, the discharged solution does not adhere to the optical window, and the like to obstruct the measurement.

Then, if required, the enclosure 26 or the pipette 15 is moved to the appropriate position, and then the computer 19 controls the pipette 15 to inject 1.5 ml of a biuret reagent (a reagent obtained by dissolving potassium sodium tartrate and copper sulfate in a sodium hydroxide solution) into the sample cell 20. Here, the computer 19 analyzes the output signal from the photosensor 18 to determine the concentration of the solution.

As described above, according to this Example, there is no need to previously weigh the volume of the solution, and by introducing the solution in an amount equal to or larger than the prescribed amount, it is possible to fix or control the volume ratio with the reagent to be injected thereinto. In this Example, only a prescribed amount of the solution is allowed to remain in the sample cell 20, and a prescribed amount of the reagent is injected, thereby fixing or controlling the volume ratio. Since this process does not involve the replacement as with Examples 1 to 3, the apparatus operates with stability even in the case where the volume ratio of the reagent is large.

Further, the apparatus is easy to transfer together with the enclosure 26 to the right place for trapping the solution to be detected, and hence it is effective. In this step, as compared with the case where only the sample cell is transferred, an optical alignment error of optical axes, and the like does not occur. Further, since the enclosure 26 has a sealed structure, there is no danger that the solution, and the like adhere to respective optical components to obstruct the measurement. Consequently, the urinalysis can be performed with ease, and further a malfunction is less likely to occur, as well as the operational stability is improved. Accordingly, the practical effect thereof is very great, and higher efficiency and laborsaving of the measurement and the test become possible.

### Example 6

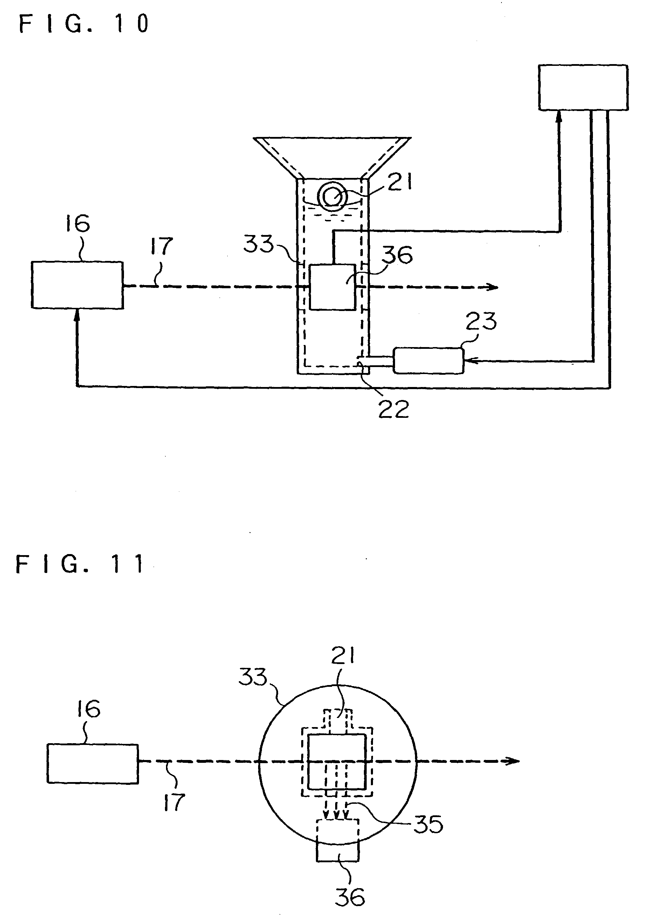

Example 6 will be described in details below by reference to FIGS. 10 and 11. FIG. 10 is a schematic view showing a configuration of a concentration measuring apparatus in accordance with this example. Whereas, FIG. 11 is a partly sectional schematic top plan view of the concentration measuring apparatus shown in FIG. 10.

In FIGS. 10 and 11, the light source 16, the photosensor 18, the computer 19, the inlet port 22, and the pipette 23 are the same as those shown in FIG. 5 in Example 2. A sample cell 33 has the form of the sample cell 10 in FIG. 5 from which either one of the outlet ports 21 has been removed. The outlet port 21 is disposed on the side having no optical window through which an irradiated light, transmitted light, and a scattered light 35 pass. Further, the output signal from a photosensor 36 disposed so as to detect the scattered light 35 is analyzed by the computer 19.

When the apparatus in accordance with this Example is used to detect the protein concentration using a urine as a solution to be detected, the operation thereof is as follows.

First, the sample cell 33 is transferred to the inside of a lavatory or the like where a urine, which is a solution to be detected, is easily trapped. Here, a subject directly discharges a urine into the sample cell 33. At this step, the urine may be discharged in any amount into the funnel-like opening of the sample cell 33 so long as it is in an amount equal to, or larger than the prescribed amount, i.e., 3 ml. The reason for this is that the surplus solution in excess of 3 ml is discharged through the outlet port 21. At this step, since the outlet port is disposed on the side having no optical window, the discharged solution does not adhere to the optical window to obstruct the measurement. Then, the sample cell 33 is returned to the original position where the optical path for the substantially parallel light 17 exists, and then the computer 19 controls the pipette 23 to inject 1.5 ml of a sulfosalicylic acid reagent (a reagent obtained by dissolving sodium sulfate in an aqueous solution of 2-hydroxy-5-sulfobenzoic acid) into the sample cell 33. Here, the computer 19 analyzes the output signal from the photosensor 36 to determine the concentration of the solution.

As described above, according to this Example, there is no need to previously weigh the volume of the solution, and by introducing the solution in an amount equal to or larger than the prescribed amount, it is possible to fix or control the volume ratio with the reagent to be injected thereinto. Consequently, the process can be simplified, and further a malfunction is less likely to occur, as well as the operational stability is improved. Accordingly, the practical effect thereof is very great, and higher efficiency and laborsaving of the measurement and the test become possible.

As described above, with the sample cell in accordance with the present invention, weighing of the solution becomes unnecessary. The practical effect thereof is very great, and higher efficiency and laborsaving of the measurement and the test become possible. Further, the sample cell, or the enclosure integrally accommodating the sample cell and respective optical components therein can be transferred to the place where the solution to be detected is easy to trap, thereby to directly charge the solution to be detected into the sample cell. Therefore, especially when the solution to be detected is a urine, the processes such as sampling and weighing can be largely simplified, and hence the practical effect thereof is very large.

Although the present invention has been described in terms of the presently preferred Examples, it is to be understood that such disclosure is not to be interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the true spirit and scope of the invention.

An object of the present invention is to eliminate a need for weighing a solution to be detected, thereby achieving higher efficiency and laborsaving of a measurement and a test, and to provide a sample cell having a configuration capable of holding the solution, and irradiating the solution with light, the sample cell, comprising: an opening for introducing the solution, and a reagent on the top side of the sample cell; and an outlet port capable of discharging a surplus solution in excess of a prescribed amount between a light propagation area in the holding portion of a sample and the opening, and a concentration measuring apparatus and a urinalysis apparatus using the same.

## Claims

1. A sample cell having a holding portion capable of holding a solution to be detected and irradiating said solution with light, said sample cell comprising: an opening for introducing said solution and a reagent on the top side of said sample cell; and an outlet port capable of discharging a surplus solution to be detected in excess of a prescribed amount between a light propagation area in said holding portion and said opening.

2. The sample cell in accordance with claim 1, wherein said opening is in funnel form.

3. The sample cell in accordance with claim 1, further comprising an inlet port for injecting said reagent at the lower part than the light propagation area in said holding portion.

4. The sample cell in accordance with claim 1, comprising: an optical entrance window for irradiating said solution to be detected with light; a first optical exit window for emitting the light transmitted through said solution; and a second optical exit window for emitting scattered light arising from said solution, said outlet port being located at a portion other than a portion having said optical window.

5. A concentration measuring apparatus of a solution. comprising: a light source for projecting light on a solution to be detected; said sample cell in accordance with claim 1 for holding said solution; a photosensor for detecting the light transmitted through said solution and/or the scattered light arising from said solution; an introduction means for introducing said solution into said sample cell; an injection means for injecting said reagent into said sample cell; and a computer for controlling said introduction means and said injection means to analyze the optical characteristics of said solution based on an output signal from said photosensor.

6. The concentration measuring apparatus in accordance with claim 5, further comprising a sealed enclosure, said light source, said sample cell, and said photosensor being integrally accommodated in said sealed enclosure, and said opening of said sample cell being open to the outside.

7. A method of urinalysis using said concentration measuring apparatus in accordance with claim 5, comprising the steps of: directly discharging a urine which is a solution to be detected from said opening of said sample cell into said holding portion; injecting a reagent from said opening or said inlet port; projecting light on said urine; and determining the transmitted light and/or the scattered light of said urine to examine said urine.

8. A sample cell having a holding portion capable of holding a solution to be detected and irradiating said solution with light, said sample cell comprising: an opening for introducing said solution, and a reagent on the top side of said sample cell; and an inclining means for inclining said sample cell.

9. The sample cell in accordance with claim 8, wherein said opening is in funnel form.

10. The sample cell in accordance with claim 8, further comprising an inlet port for injecting said reagent at the lower part than the light propagation area in said holding portion.

11. The sample cell in accordance with claim 8, comprising: an optical entrance window for irradiating said solution with light; a first optical exit window for emitting the light transmitted through said solution; and a second optical exit window for emitting the scattered light arising from said solution, said outlet port being located at a portion other than a portion having said optical window.

12. A concentration measuring apparatus of a solution, comprising: a light source for projecting light on a solution to be detected; said sample cell in accordance with claim 7 for holding said solution; a photosensor for detecting the light transmitted through said solution and/or the scattered light arising from said solution; an introduction means for introducing said solution into said sample cell; an injection means for injecting said reagent into said sample cell; and a computer for controlling said inclining means, said introduction means, and said injection means to analyze the optical characteristics of said solution based on an output signal from said photosensor.

13. The concentration measuring apparatus of a solution in accordance with claim 12, further comprising a sealed enclosure, said light source, said sample cell, and said photosensor being integrally accommodated in said sealed enclosure, and said opening of said sample cell being open to the outside.

14. A method of urinalysis using said concentration measuring apparatus in accordance with claim 12, comprising the steps of: directly discharging a urine which is a solution to be detected from said opening of said sample cell into said holding portion; discharging said surplus solution to be detected in excess of a prescribed amount through said opening by inclining said sample cell by said inclining means; injecting a reagent from said opening or said inlet port; and determining the transmitted light and/or scattered light of said urine to examine said urine.
